# EUROPÄISCHE PATENTSCHRIFT

(11) **EP 2 920 721 B1**
(45) Veröffentlichungstag und Bekanntmachung des Hinweises auf die Patenterteilung: **27.03.2019**
(21) Anmeldenummer: 13798275.7
(22) Anmeldetag: 13.11.2013
(51) Int. Cl.: G16H 40/63

(54) **MEDIZINISCHES STEUERUNGSSYSTEM**
MEDICAL CONTROL SYSTEM
SYSTÈME DE COMMANDE MÉDICAL

(30) Priorität: 13.11.2012 DE 102012220672
(43) Veröffentlichungstag der Anmeldung: 23.09.2015
(73) Patentinhaber: TRUMPF Medizin Systeme GmbH + Co. KG, 07318 Saalfeld (DE)
(72) Erfinder: RUDOLF, Marka, 85737 Ismaning (DE); ÖZHAN, Serhan, 81379 München (DE); SCHWEBEL, Tim, 82266 Inning (DE); HAN, Gel, 80802 München (DE)
(74) Vertreter: Prüfer & Partner mbB Patentanwälte · Rechtsanwälte
(86) Internationale Anmeldenummer: PCT/EP2013/073750
(87) Internationale Veröffentlichungsnummer: WO 2014/076141

(56) Entgegenhaltungen:
- EP-A2- 1 181 897
- EP-A2- 1 677 224
- US-A1- 2002 111 701
- "Tegris Redefining or Integration", , 1. Oktober 2012 (2012-10-01), XP055129429, Rastatt, Germany Gefunden im Internet: URL:http://www.maquet.com/content/Surgical Workplaces/Documents/Brochures/Internet_TE GRIS_Brochure_10000911-EN-1B_NONUS.pdf [gefunden am 2014-07-16]
- "SWITCHPOINT INFINITY(TM) CONTROL SYSTEM OPERATIONS AND MAINTENANCE MANUAL", , 1. Januar 2004 (2004-01-01), XP055128973, Gefunden im Internet: URL:http://aamedicalstore.com/media/manual s/Stryker Switchpoint Infinity Manual.pdf [gefunden am 2014-07-15]

## Beschreibung

Die Erfindung betrifft ein medizinisches Steuerungssystem und insbesondere ein medizinisches Steuerungssystem in Verbindung mit einem Bediengerät mit einem Touchscreen.

Es sind medizinische Steuerungssysteme bekannt, die in der Lage sind, mehrere verschiedene Medizingeräte anzusteuern. In der Druckschrift EP 1 394 717 B1 ist beispielsweise ein Touchscreen offenbart, auf dem Abbildungen der Bedienoberflächen der verschiedenen Medizingeräte dargestellt werden.

Dabei besteht das Problem, dass das Bedienkonzept der Bedienoberflächen der verschiedenen Medizingeräte übernommen wird, wobei keine einheitliche Bedienung gewährleistet ist.

Druckschrift EP 1 181 897 A2 offenbart eine chirurgische Navigations- und Bildgebungs-Workstation, die Eingänge für Verfolgungssensoren von chirurgischen Werkzeugen oder Geräten aufweist, wobei eine Position des chirurgischen Werkzeugs oder der Geräte erfasst wird.

Der Erfindung liegt die Aufgabe zugrunde, ein medizinisches Steuerungssystem bereitzustellen, das eine intuitive Bedienung von verschiedenen Medizingeräten über eine jeweilige Bedienoberfläche mit einem einheitlichen Erscheinungsbild und einem einheitlichen Bedienkonzept ermöglicht.

Die Aufgabe wird durch ein medizinisches Steuerungssystem gemäß Anspruch 1 oder 2 und ein Verfahren gemäß Anspruch 7 gelöst. Weiterentwicklungen der Erfindung sind Gegenstand der Unteransprüche.

Durch das medizinische Steuerungssystem gemäß einem Aspekt der Erfindung werden im Wesentlichen gleiche spezifische Bedienfunktionen der verschiedenen Medizingeräte mit jeweils im Wesentlichen gleichen

Symbolen dargestellt, wodurch eine einfache und intuitive Bedienung der Medizingeräte möglich ist.

Gemäß einem weiteren Aspekt werden die Medizingeräte so dargestellt, dass die Anordnung ihrer dargestellten Positionen der Anordnung aus einer Sicht des Bediengeräts entspricht, wodurch eine einfache und intuitive Bedienung der Medizingeräte möglich ist.

Die Erfindung wird nun anhand von Ausführungsbeispielen unter Bezugnahme auf die beigefügten Zeichnungen erläutert.

Insbesondere zeigen:
- Fig. 1: ein medizinisches Steuerungssystem;
- Fig. 2: eine erste Anzeige eines Touchscreens eines Bediengeräts mit mehreren Medizingeräten;
- Fig. 3: eine zweite Anzeige des Touchscreens mit einer Darstellung von einem Operationstisch als eines der mehreren Medizingeräte und von Symbolen für spezifische Bedienfunktionen;
- Fig. 4: eine dritte Anzeige des Touchscreens mit einer Darstellung des einen Medizingeräts und eines Zeigers für eine Bewegungsrichtung und eine mögliche Bewegungsgeschwindigkeit;
- Fig. 5: die dritte Anzeige des Touchscreens mit der Darstellung des einen Medizingeräts und eines Zeigers für eine andere Bewegungsrichtung und eine mögliche Bewegungsgeschwindigkeit als in Fig. 4;
- Fig. 6: die dritte Anzeige des Touchscreens mit einer Darstellung des einen Medizingeräts sowie von Endlagen von angetriebenen Komponenten von dem Medizingerät und einer möglichen Bewegungsgeschwindigkeit der angetriebenen Komponenten in die Endlagen;
- Fig. 7.: die zweite Anzeige des Touchscreens mit einer Darstellung von einer Operationsleuchte als eines der mehreren Medizingeräte sowie von Symbolen für spezifische Bedienfunktionen;
- Fig. 8: eine Draufsicht auf Operationssäle mit einer Anordnung der Medizingeräte mit Tags und einer Anordnung von Lesemodulen für eine genaue Positionsbestimmung der Medizingeräte;
- Fig. 9: eine Draufsicht auf die Operationssäle mit einer Anordnung der Medizingeräte mit den Tags und einer Anordnung der Lesemodule für eine einfachere Positionsbestimmung; und
- Fig. 10: einen Startbildschirm mit einer schematischen Anordnung der Medizingeräte mit den Tags.

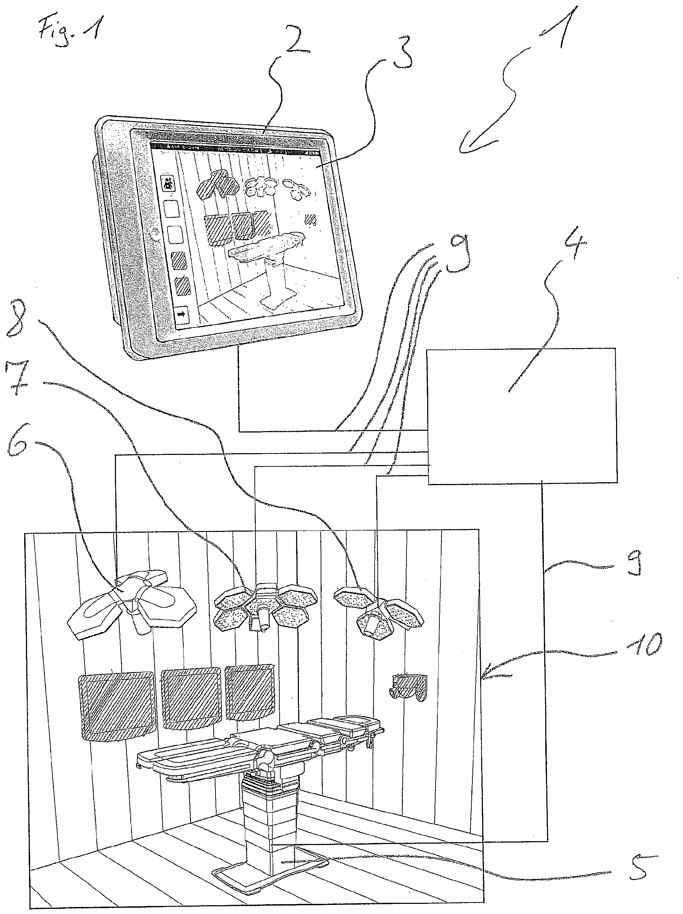

Fig. 1 zeigt ein medizinisches Steuerungssystem 1. Das medizinische Steuerungssystem 1 weist ein Bediengerät 2 in Form eines Tablet-Computers mit einem Touchscreen 3, eine Steuerungsvorrichtung 4 und mehrere Medizingeräte in Form eines Operationstischs 5, und Operationsleuchten 6, 7, 8 in einem Operationssaal 10 auf. Die Medizingeräte 5, 6, 7, 8 sind mittels Datenverbindungen 9 mit der Steuerungsvorrichtung 4 verbunden. Die Steuerungsvorrichtung 4 kann Teil des Bediengeräts selbst oder auch von einer separaten Einrichtung sein.

Die Ausstattung des medizinischen Steuerungssystems 1 in dieser Ausführungsform ist beispielhaft. In alternativen Ausführungsformen sind eine andere Anzahl, andere Arten und eine andere Anordnung der Medizingeräte 5, 6, 7, 8 möglich. Auch ist das medizinische Steuerungssystem 1 nicht auf einen Operationssaal 10 beschränkt, sondern kann alternativ auch in anderen Eingriffsräumen oder auf einer Intensivstation verwendet werden.

Das Bediengerät 2 dient dazu, zusätzlich oder alternativ zu den an den Medizingeräten 5, 6, 7, 8 vorgesehenen Bedieneinrichtungen, eine stationäre oder optional mobile Bedieneinheit zu schaffen. Für eine stationäre Ausführung ist das Bediengerät 2 beispielsweise an einer Wand befestigt, oder in einer Dockingstation aufgenommen, die ebenfalls an der Wand oder beispielsweise an einer medizinischen Versorgungseinheit befestigt ist. Im Falle der Dockingstation ist es möglich, eine Datenverbindung 9 über die Dockingstation herzustellen und das Bediengerät 2 mit elektrischer Energie zu versorgen. Ebenfalls besteht die Möglichkeit, das Bediengerät 2 aus der Dockingstation zu entnehmen und mobil zu verwenden.

Die Datenverbindung 9 kann über Kabel, oder alternativ drahtlos, beispielsweise über Funk oder Infrarot, erfolgen.

Das Bediengerät 2 kann entweder im unsterilen Bereich oder mit einer entsprechenden sterilisierbaren Ausstattung auch im sterilen Bereich des Operationssaals 10 betrieben werden.

Der in Fig. 1 gezeigte Touchscreen 3 zeigt eine Darstellung des Operationssaals 10 und eine schematische Darstellung der Medizingeräte 5, 6, 7, 8. Die mit dem Bediengerät 2 nicht bedienbaren Medizingeräte 5, 6, 7, 8, hier die Operationsleuchte 6, sind auf dem Touchscreen 3 abgedunkelt dargestellt.

Die Ausstattung des aktuellen Operationssaals 10 wird durch einen Lokalisierungsdienst in einem übergeordneten System erkannt, und alle im Raum befindlichen Medizingeräte 5, 6, 7, 8 werden auf einem Startbildschirm auf dem Touchscreen 3 angezeigt. Dadurch sieht ein Benutzer die im Operationssaal 10 befindlichen Medizingeräte 5, 6, 7, 8. Alternativ ist es auch möglich, die Medizingeräte 5, 6, 7, 8 benutzerspezifisch oder anwendungsspezifisch, also für bestimmte Operationen, anzuzeigen oder zu bedienen. Die erkannten Geräte können alternativ auch entweder in einer entsprechend einem vorkonfigurierten Benutzerbildschirm, oder in einer, wie nachstehend erläuterten, tatsächlichen durch den Lokalisierungsdienst erkannten, oder in einer darin oder in dem medizinischen Steuerungssystem 1 hinterlegten, Anordnung angezeigt werden.

Des Weiteren ist alternativ auch eine dynamische Anzeige der Medizingeräte möglich. Bei einer Veränderung der Position der Medizingeräte werden die aktuellen Positionen auf dem Touchscreen angezeigt, so dass eine gegebenenfalls aktuell aufgetretene Kollisionsgefahr erkannt werden kann.

Die Darstellung des Operationssaals 10 ist in einer alternativen Ausführungsform so vorgesehen, dass die Medizingeräte 5, 6, 7, 8 in vordefinierten Positionen in einem Raum angeordnet sind. Die Medizingeräte 5, 6, 7, 8 werden in den vordefinierten Positionen dargestellt. Die Darstellung des Operationssaals 10 ist dreidimensional, kann aber alternativ auch zweidimensional sein. In einer weiteren alternativen Ausführungsform ist die Darstellung dynamisch, so dass die Darstellung auf dem Touchscreen 3 des Bediengeräts 2 so verändert wird, dass die Darstellung der Medizingeräte 5, 6, 7, 8 einer Sicht auf die Medizingeräte 5, 6, 7, 8 von dem Bediengerät 2 aus entspricht.

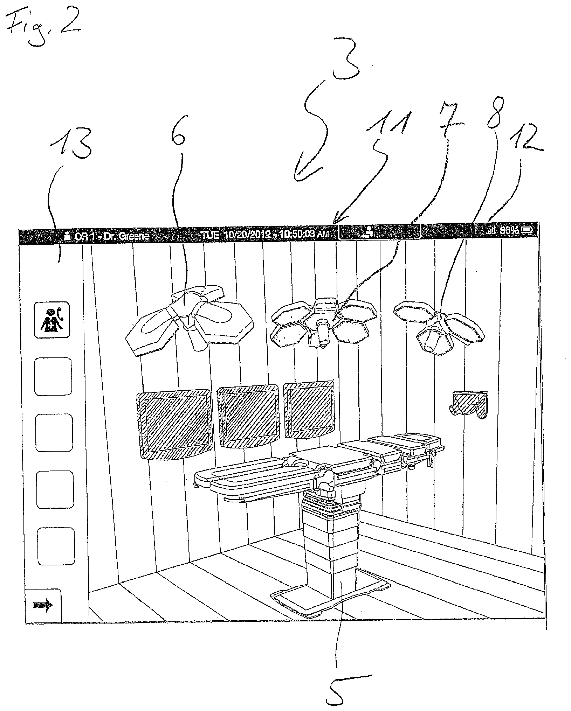

In Fig. 2 ist der Startbildschirm auf dem Touchscreen 3 als eine erste Anzeige 11 gezeigt. Die erste Anzeige 11 zeigt die mit der Steuerungsvorrichtung 4 verbundenen und durch das Bediengerät 2 bedienbaren Medizingeräte 5, 6, 7, 8 in Form des Operationstischs 5 und der Operationsleuchten 6, 7, 8 an. Weitere Geräte, wie Monitore und eine Kamera, sind abgedunkelt dargestellt und sind somit erkennbar nicht durch das Bediengerät 2 bedienbar. Des Weiteren können alternativ auch von dem Lokalisierungsdienst erkannte und angezeigte Medizingeräte, beispielsweise auf Grund einer Inkompatibilität, nicht durch das medizinische Steuerungssystem 1 ansteuerbar sein. Diese Medizingeräte 5, 6, 7, 8 sind ebenfalls abgedunkelt dargestellt.

Weiterhin sind auf der ersten Anzeige 11, wie auch auf weiteren Anzeigen, eine Statuszeile 12 dargestellt und eine Favoritenleiste 13 ist anzeigbar.

In der Statuszeile 12 ist hier die Nummer des Operationssaals 10 ("OR 1"), der aktuelle Benutzer des medizinischen Steuerungssystems 1 ("Dr. Greene") und das aktuelle Datum und die aktuelle Uhrzeit dargestellt. Ebenso ist ein "Abmelde-Button" zum Abmelden aus dem medizinischen Steuerungssystem 1 vorhanden und eine Anzeige eines Ladezustands eines Akkumulators des Bediengeräts 2 vorgesehen.

In der Favoritenleiste 13 ist hier ein Button für "Springer rufen" gezeigt. Benutzer- oder anwendungsspezifisch können hier weitere Favoriten-Button angezeigt und betätigt werden.

Im Betrieb wird durch Berühren der Darstellung von einem der Medizingeräte 5, 6, 7, 8 auf der ersten Anzeige 11 des Touchscreens 3 eine weitere Anzeige aufgerufen, in der spezifische Bedienfunktionen von dem auf dem Touchscreen 3 berührten Medizingerät 5, 6, 7, 8 angezeigt werden. Im vorliegenden Fall wird beispielhaft die schematische Darstellung des Operationstischs 5 auf dem Touchscreen 3 berührt.

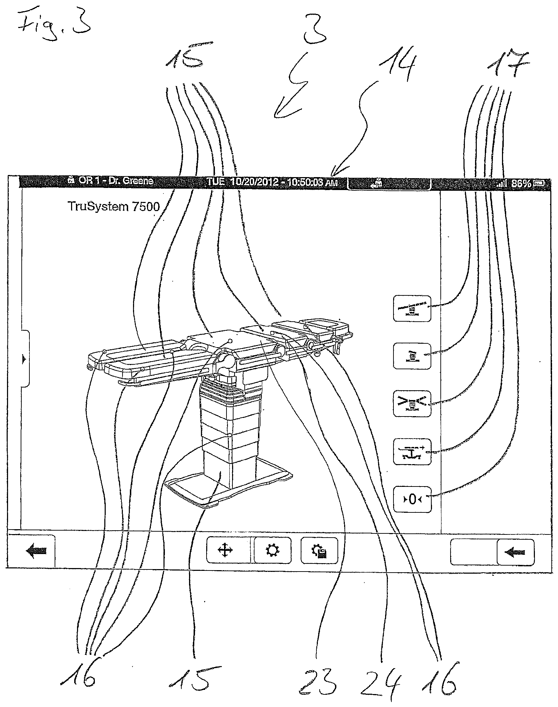

In Fig. 3 ist die weitere Anzeige als eine zweite Anzeige 14 optional auf dem Touchscreen 3 dargestellt. In der zweiten Anzeige 14 sind angetriebene Komponenten 15 des Operationstischs 5 dargestellt. Die angetriebenen Komponenten 15 oder Gruppierungen der angetriebenen Komponenten 15 sind mit kleinen Kreisen 16 markiert, um die angetriebenen Komponenten 15 mit spezifischen Bedienfunktionen anzusprechen. Des Weiteren sind optional Symbole 17 für weitere spezifische Bedienfunktionen, nämlich für ein Bewegen von Gruppierungen der angetriebenen Komponenten 15 in vorbestimmte Richtungen als die spezifische Bedienfunktion, die auf Grund ihrer Komplexität nicht mehr durch Anwählen einzelner angetriebener Komponenten 15 selektiert werden kann, angezeigt. Beispielsweise ist hier das Anfahren einer Trendelenburgstellung durch das oberste Symbol 17 und das Anfahren einer Nullstellung durch das unterste Symbol 17 angegeben, so das im Wesentlichen gleiche Bedienfunktionen mit im Wesentlichen gleichen Symbolen dargestellt werden.

Im Betrieb wird durch Berühren des Kreises auf der Darstellung von einer der angetriebenen Komponenten 15 eine weitere Anzeige aufgerufen bzw. die Darstellung funktionsspezifisch erweitert, auf der eine mögliche Bewegungsrichtung und Vorgaben für ihre mögliche Bewegungsgeschwindigkeit angezeigt werden. Im vorliegenden Fall wird beispielhaft der Kreis auf dem Operationstischplattensegment 23 ("Unterer Rücken") berührt.

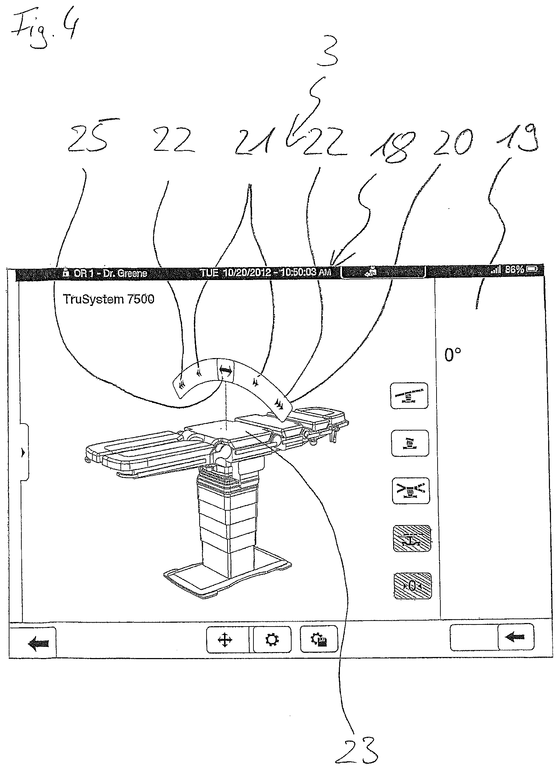

In Fig. 4 wird die nun weitere Anzeige als eine dritte Anzeige 18 auf dem Touchscreen 3 dargestellt. Oberhalb des Operationstischplattensegments 23 ist nun ein Kreisbogensegment 20 angezeigt, das die Bewegungsrichtung der spezifischen Bedienfunktion des Operationstischs 5, hier eine Kantung, zeigt. Im Verlauf des Kreisbogensegments 20 sind von seiner Mitte ausgehend in beide Richtungen jeweils ein Doppelpfeil 21 und ein Dreifachpfeil 22 gezeigt. Die Pfeile 21, 22 zeigen eine mögliche Bewegungsgeschwindigkeit der spezifischen Bedienfunktion an.

In einer Informationsspalte 19 ist hier optional ein Kantungswinkel (hier: 0°) als Information einer momentanen Position in der möglichen Bewegungsrichtung angezeigt.

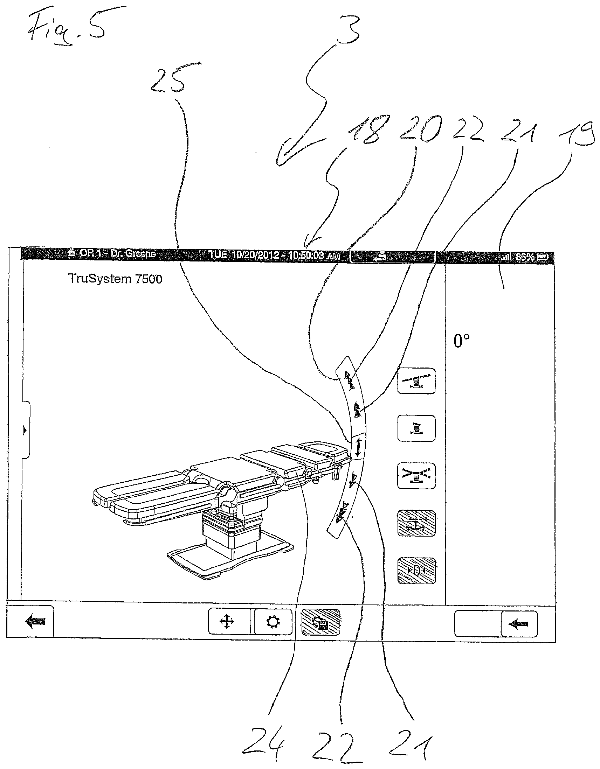

In Fig. 5 wird eine weitere auf dem Touchscreen 3 dargestellte dritte Anzeige 18 gezeigt. Der Unterschied zu der in Fig. 4 gezeigten Anzeige liegt darin, dass durch ein vorangehendes Berühren des Kreises 16 auf dem Operationstischsegment 24 ("Rücken") auf der zweiten Anzeige 14 (Fig. 3) die spezifische Bedienfunktion für die Bewegung des Operationstischsegments 24 ("Rücken") gezeigt wird. Hier wird als die mögliche Bewegungsrichtung ein Schwenken der oberen drei Operationstischplattensegmente durch das entsprechenden Kreissegment 20 und die mögliche Geschwindigkeit durch die Doppelpfeile 21 und Dreifachpfeile 22 angezeigt.

Die Bedienfunktionen in Fig. 4 und Fig. 5 werden also mit gleichen Symbolen dargestellt, so dass im Wesentlichen gleiche Bedienfunktionen durch im Wesentlichen gleiche Symbole dargestellt werden.

Im Betrieb werden die Bedienfunktionen durch ein Berühren eines Doppelpfeilzeigers 25 und durch ein Verfahren während der Berührung entlang des Kreisbogensegments 20 aktiviert. Die Geschwindigkeit der Bedienfunktion ist von dem Ausmaß des Verfahrens entlang des Kreisbogens 22 abhängig. Je weiter das Verfahren entlang des Kreisbogens 22 erfolgt, umso schneller wird die Bedienfunktion ausgeführt.

In der Informationsspalte 19 wird hier der Schwenkwinkel des Operationstischplattensegments 24, also die momentane Position in der möglichen Bewegungsrichtung, angezeigt.

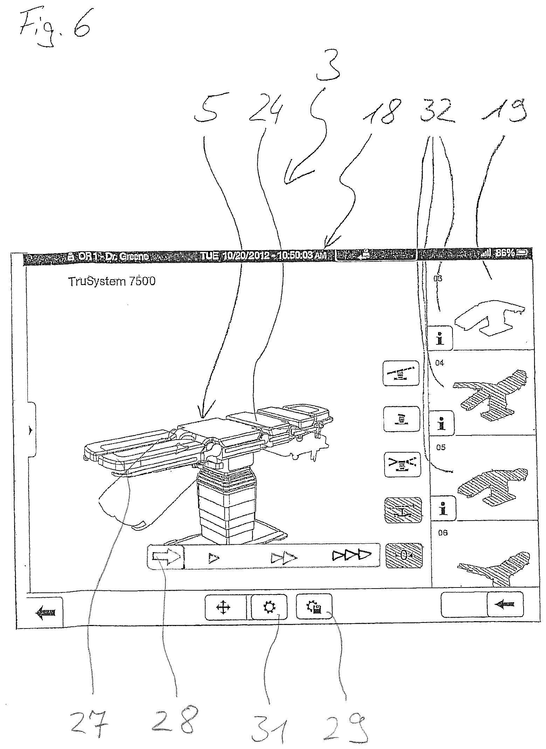

In Fig. 6 zeigt die dritte Anzeige 18 auf dem Touchscreen 3 optional den Operationstisch 5, wobei Endlagen der angetriebenen Komponenten 15 und eine mögliche Bewegungsgeschwindigkeit durch den Doppelpfeil 21 und den Dreifachpfeil 22 in einem Balken 26 dargestellt sind.

Durch ein Berühren einer Preset-Taste 31 werden optional mehrere Preset-Symbole 32 in der Informationsspalte 19 dargestellt, über die die Endlagen für die angetriebenen Komponenten 15 auf dem Touchscreen 3 als verschiedene vorbestimmte Positionen dargestellt werden und angefahren werden können. Die angetriebenen Komponenten 15 sind hier Beinplatten 27 und das Operationstischsegment 24.

Im Betrieb wird hier das Anfahren der vorbestimmten Positionen durch Berühren von einem der Preset-Symbole 32 ermöglicht. Ein Verfahren der angetriebenen Komponenten 15 wird dann durch Berühren eines Pfeilzeigers 28 und dessen Verfahren während der Berührung entlang des Balkens 26 aktiviert. Die Geschwindigkeit der Bedienfunktion ist von dem Ausmaß des Verfahrens entlang des Balkens 26 abhängig. Je weiter das Verfahren entlang des Balkens 26 erfolgt, umso schneller wird die Bedienfunktion ausgeführt.

Die Bedienfunktion einer Vorgabe der Bewegungsgeschwindigkeit für das Anfahren von vorbestimmten Positionen ist also im Wesentlichen gleich der wie in Fig. 4 und Fig. 5 gezeigten Vorgabe für die Bewegungsgeschwindigkeit der Bewegung eines Tischplattensegments, so dass im Wesentlichen gleiche Bedienfunktionen durch im Wesentlichen gleiche Symbole dargestellt werden.

Ferner ist in der dritten Anzeige 18 optional ein Abspeichersymbol 29 vorgesehen.

Im Betrieb speichert die medizinische Steuerungsvorrichtung 1 bei einem Berühren des Abspeichersymbols 29 die jeweils momentane Position der angetriebenen Komponenten 15 ab. Dabei wird optional ein weiteres Preset-Symbol 32 in der Informationsspalte 19 erzeugt. Das erzeugte Preset-Symbol 32 entspricht einem Abbild der abgespeicherten Position, also der durch die Preset-Auswahl gewünschten Endposition des Operationstisches 5, wenn dieses abgespeicherte Preset aufgerufen wird. Neben dem Erreichen einer Endposition des Operationstisches 5 kann ein Preset auch dazu genutzt werden, eine gewünschte Gruppierung von Komponenten 15 in einem vordefinierten Verhältnis zueinander zu bewegen.

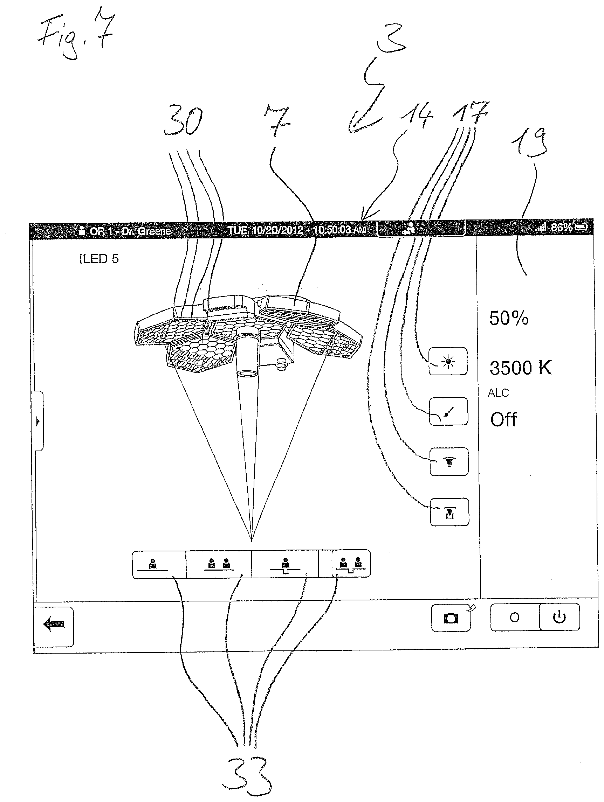

In Fig. 7 ist die zweite Anzeige 14 des Touchscreens 3 mit einer Darstellung der Operationsleuchte 7 als eines der mehreren Medizingeräte 5, 6, 7, 8 und von spezifischen Bedienfunktionen gezeigt. Die Operationsleuchte 7 weist Beleuchtungskomponenten 30 auf, die ein Leuchtfeld auf einer Operationsstelle bilden. In alternativen Ausführungsformen sind die Beleuchtungskomponenten 30 für eine Allgemeinbeleuchtung des Operationssaals 10 oder eines anderen Raums vorgesehen.

Des Weiteren sind optional die Symbole 17 für die spezifischen Bedienfunktionen der Operationsleuchte 7 dargestellt. Die spezifischen Bedienfunktionen sind hier entsprechend den Symbolen von oben nach unten eine Helligkeitseinstellung, eine Farbtemperatureinstellung, eine Einstellung einer Leuchtfeldgröße und eine Auswahl von betriebenen Beleuchtungskomponenten 30. Durch Berühren der Symbole 17 für die spezifischen Bedienfunktionen werden Symbole 33 für einstellbare Parameter dieser spezifischen Bedienfunktion gezeigt.

In Fig. 7 ist auf der zweiten Anzeige 14 die spezifische Funktion der "Auswahl von betriebenen Beleuchtungskomponenten" ausgewählt. Dabei werden die Symbole 33 für die einstellbaren Parameter angezeigt. Die Auswahl der betriebenen Beleuchtungskomponenten ist hier gemäß den Symbolen 33 abhängig von den Parametern, ob ein oder mehrere Operateure operieren, und ob es eine flache oder eine tiefe Operationswunde ist.

In der Informationsspalte 19 sind die momentanen Einstellungen von Parametern, nämlich 50% der Helligkeit, eine Farbtemperatur von 3500 K, und dass die Autofocusfunktion (ALC) ausgeschaltet ist, angezeigt.

Im Betrieb wird eines der Symbole 17 für die spezifischen Bedienfunktionen auf dem Touchscreen 3 berührt und dadurch werden die Symbole 33 für einstellbare Parameter angezeigt. Durch Berühren von einem der Symbole 33 für einstellbare Parameter werden die entsprechenden Beleuchtungskomponenten 30 der Operationsleuchte 7 angesteuert, um die ausgewählte spezifische Bedienfunktion auszuführen.

Durch die im Wesentlichen gleiche Bedienabfolge und die im Wesentlichen gleiche Darstellung der Art der Bedienelemente des medizinischen Steuerungssystems ist die Bedienkonzeption für sämtliche Medizingeräte einheitlich, so dass die Bedienung vereinfacht und sicher durchgeführt werden kann.

Die sichere und vereinfachte Bedienung wird dadurch unterstützt, dass eine Darstellung der Anordnung der Medizingeräte auf dem Bediengerät (2) der Anordnung der Medizingeräte (5, 6, 7, 8) in dem Raum aus einer Sicht des Bediengerätes (2) entspricht. Dadurch ist eine einfache und intuitive Bedienung der Medizingeräte (5, 6, 7,8) möglich.

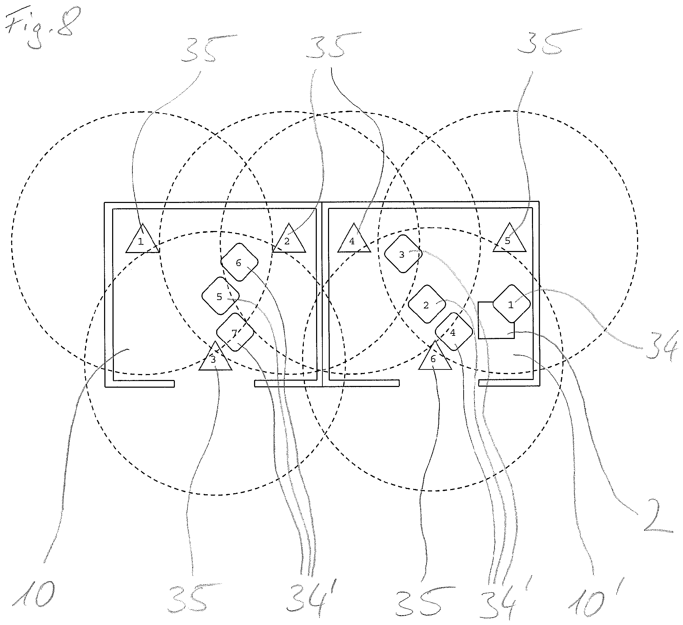

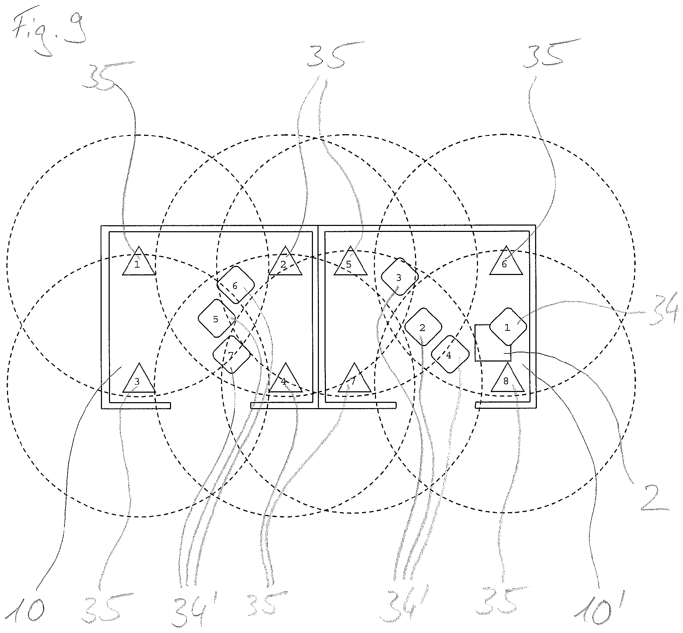

Eine Lokalisierung des Bediengeräts 2 und der Medizingeräte ist grundsätzlich in den Fig. 8 und 9 gezeigt.

Fig. 8 und 9 zeigen jeweils eine Draufsicht auf zwei Operationssäle 10, 10' mit einer Anordnung des Bediengeräts 2, der Medizingeräte und einer Anordnung von Lesemodulen 35 für eine genaue Positionsbestimmung der Medizingeräte und des Bediengeräts 2.

Das Bediengerät 2 ist mit einem Bluetooth-Low-Energy-Sendemodul, einer ersten Sendeeinheit oder sogenannten Tag 34 versehen und die Medizingeräte sind mit einer zweiten Sendeeinheit oder sogenannten Tag 34' versehen. Das Tag 34 des Bediengeräts 2 ist in einem separaten Gehäuse, in dem das Bediengerät aufgenommen ist, oder alternativ in dem Bediengerät 2 selbst enthalten. Sämtliche Medizingeräte, die auf dem Bediengerät 2, beispielsweise auf einem Startbildschirm, angezeigt werden sollen, sind mit einem Tag 34' versehen. Die Tags 34, 34' senden in periodischen Abständen, also zyklisch, Identifikationsinformationen zu ihrer Identifikation als elektromagnetische Strahlung, hier einem Funksignal, aus. Beispielsweise wird eine MAC-Adresse oder eine Name abgegeben. Alternativ erfolgt das Senden von Informationen nicht zyklisch, sondern kontinuierlich, oder beispielsweise nur bei einer erkannten Bewegung des Bediengeräts 2 oder des Medizingeräts.

In den Operationssälen 10, 10' sind die Lesemodule 35 vorgesehen. Die Lesemodule 35 sind an der Raumdecke oder alternativ an Wänden der Operationssäle 10, 10' vorgesehen. Die Lesemodule 35 erfassen die Stärke der elektromagnetische Strahlung, beispielsweise ein Funksignal, der jeweiligen Tags 34, 34', wirken hier als ein Bluetooth-Low-Energy-Lesemodul, und geben diese über eine Datenverbindung an die Steuerungsvorrichtung 4 weiter. Alternativ können auch Infrarotstrahlen von den Sendeeinheiten ausgestrahlt und von den Lesemodulen empfangen werden, oder die Sendeeinheiten als Ultra-Breitband-Sendemodule und die Lesemodule als Ultra-Breitband-Lesemodule ausgebildet sein. In einer weiteren Alternative kann eine Verteilung der Geräte mittels optischem Tracking festgestellt werden.

Diese Lesemodule 35 sind in dem Raum jeweils an vorbestimmten Positionen angeordnet. Das medizinische Steuerungssystem 1 ist so angepasst, über die erfasste Stärke der Signale der Tags 34, 34', wie nachstehend erläutert, relative Positionen der Medizingeräte mit den Tags 34' zu dem Bediengerät 2 mit dem Tag 34 zu bestimmen und an das Bediengerät 2 zu übermitteln.

Das Bediengerät 2 ist so angepasst, dass es, von der Steuerungsvorrichtung 4 angesteuert, die schematische Darstellung der Medizingeräte entsprechend ihren relativen Positionen zu dem Bediengerät 2 anzeigt, so dass die Anordnung von den auf dem Bediengerät 2 dargestellten Positionen der Medizingeräte der Anordnung der Medizingeräte aus der Sicht aus der Position des Bediengeräts 2 entspricht.

In Fig. 8 sind in einem der Operationssäle 10, 10' jeweils drei Lesemodule 35 angeordnet. Die mit gestrichelten Linien dargestellten Kreise um die Lesemodule 35 zeigen Empfangsräume, in denen die Lesemodule 35 die Signale der Tags 34, 34' empfangen, wobei die gestrichelten Linien diese Empfangsräume nicht begrenzen.

Ferner sind in Fig. 8 in jedem der Operationssäle 10, 10' jeweils drei Tags 34' gezeigt, die jeweils an einem der Medizingeräte angebracht sind. In einem der Operationssäle 10' ist ferner das Bediengerät 2 mit dem Tag 34 gezeigt. Die Positionen der Tags 34' an den Medizingeräten und des Tags 34 an dem Bediengerät 2, und somit die relative Position der Medizingeräte zu dem Bediengerät 2, werden durch das medizinische Steuerungssystem 1 über eine Triangulation, also über eine Erfassung der Signalstärken an den Lesemodulen 35 und daraus folgend einer Bestimmung der Position der Tags 34, 34' bestimmt.

In Fig. 9 ist eine einfachere Positionsbestimmung gezeigt. In der Draufsicht auf die Operationssäle 10, 10' sind das Tag 34 an dem Bediengerät 2 und die Tags 34' an den Medizingeräten an den gleichen Positionen wie in Fig. 8 gezeigt. In Fig. 9 sind jedoch in jedem der Operationssäle 10, 10' vier Lesemodule 35 angeordnet. Die mit gestrichelten Linien dargestellten Kreise um die Lesemodule 35 zeigen auch hier Empfangsräume, in denen die Lesemodule 35 die Signale der Tags 34, 34' empfangen. Auch hier begrenzen die gestrichelten Linien diese Empfangsräume nicht. Die Operationssäle 10, 10' weisen, wie nachstehend beschrieben, verschiedene Bereiche auf, die die die Lesemodule 35 umgeben, und die den Lesemodulen 35 zugeordnet sind. Die Position der Tags 34, 34', und somit die relative Position der Medizingeräte zu dem Bediengerät 2, werden über eine Erfassung der Bereiche der Operationssäle 10, 10', in denen sich die Tags 34, 34' befinden, durch das medizinische Steuerungssystem 1 bestimmt. Der Bereich, der demjenigen Lesemodul 35 zugeordnet ist, das einen höchsten Signalstärkewert des Tags 34 des Bediengeräts 2 empfängt, ist der Bereich, in dem sich der Benutzer mit dem Bediengerät aktuell aufhält. Alternativ oder ergänzend kann auch das Signal weiterer Bereiche ausgewertet werden, wenn sich beispielsweise das Tag 34, 34' zwischen zwei Bereichen der Räume befindet, oder mehrere Tags in einem Bereich sind.

In einer alternativen Ausführungsform ist das Bediengerät nicht so ausgebildet, dass auf den Startbildschirm mit den Medizingeräten als der ersten Anzeige bei Betätigung eine zweite Anzeige die Medizingeräte mit den Symbolen für die Bedienfunktionen angezeigt wird. Vielmehr sind die Bedienfunktionen der Medizingeräte in dieser Ausführungsform bereits auf dem Startbildschirm angezeigt und können dort betätigt werden. Alternativ ist die Anzeige mit der Anordnung der Medizingeräte nicht der Startbildschirm, sondern wird durch eine vorbestimmte Eingabe aufgerufen.

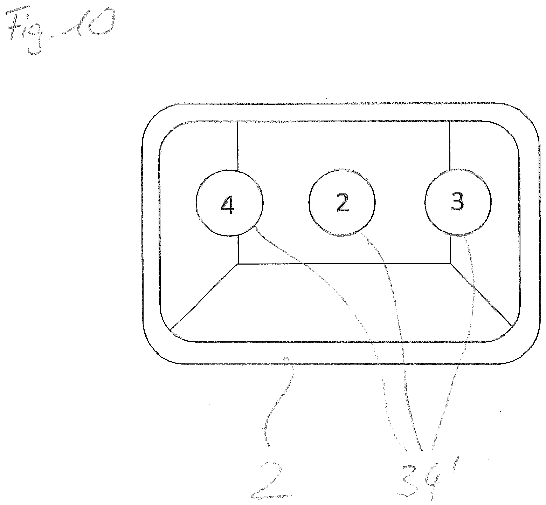

Im Betrieb senden das Tag 34 des Bediengeräts 2 und die Tags 34' der Medizingeräte mittels elektromagnetischer Strahlen, hier Bluetooth-Low-Energy, jeweils Informationen, z.B. ihre MAC-Adresse oder ihren Namen. Diese Informationen werden von den Lesemodulen 35 empfangen. Die Signalstärke der jeweiligen elektromagnetischen Strahlen der Tags 34, 34' werden an den Lesemodulen 35 gemessen und das medizinische Steuerungssystem 1 bestimmt über die Signalstärken mittels einer Triangulation die relativen Positionen der Tags 34', und damit der Medizingeräte, zu dem Tag 34 an dem Bediengerät 2. Diese relativen Positionen werden an das Bediengerät 2 übermittelt und das Bediengerät 2 zeigt auf einem Startbildschirm die schematische Darstellung der Medizingeräte entsprechend den relativen Positionen (siehe Fig. 10). Somit entspricht eine Anordnung von auf dem Bediengerät 2 dargestellten Positionen der Medizingeräte einer Anordnung der Medizingeräte aus einer Sicht aus einer Position des Bediengeräts 2. Dabei wird angenommen, dass ein Benutzer des Bediengeräts 2 mit dem Rücken zu einer Wand des Raums steht und seinen Blick zur Raummitte, also zu den Medizingeräten gerichtet hat.

Durch die Triangulation der Signalstärken mit Hilfe der in Fig. 8 gezeigten Konfiguration ist es möglich, die genaue Position der Tags 34, 34' und damit ihre relative Position zueinander zu bestimmen. Über die erfasste Signalstärke wird jeweils der Abstand eines bestimmten Tags 34, 34' von den drei Lesemodulen 35 bestimmt. Durch die Auswertung der Abstände des bestimmten Tags 34, 34' von den drei Lesemodulen 35, in der jeweils quasi ein Kreis mit dem erfassten Abstand um die Lesemodule 35 gelegt wird, wobei die Position des bestimmten Tags 34, 34' in dem Schnittpunkt dieser Kreise liegt, und die Kenntnis der Position der Lesemodule wird die Position des bestimmten Tags 34, 34' bestimmt. Das Medizingerät mit dem Tag 34' "4" wird also auf dem Bediengerät 2 links, das Medizingerät mit dem Tag 34' "2" auf dem Bediengerät 2 mittig und das Medizingerät mit dem Tag 34' "3" auf dem Bediengerät 2 rechts dargestellt (siehe Fig. 10).

Mit der in Fig. 9 gezeigten Konfiguration ist es möglich, festzustellen, in welchem Bereich in den Operationssälen 10, 10' sich eines der Tags 34, 34' befindet. Die Tags 34, 34' können den Bereichen "links oben" (Lesemodul 35 "1", Lesemodul 35 "5"), rechts oben" (Lesemodul 35 "2", Lesemodul 35 "6"), "links unten" (Lesemodul 35 "3", Lesemodul 35 "7") und "rechts unten" (Lesemodul 35 "4", Lesemodul 35 "8") angeordnet sein. Hier befinden sich Tag 34 "1" "rechts unten" in der Nähe von Lesemodul 35 "8", Tag 34' "2" "links unten" in der Nähe von Lesemodul 35 "7" und Tag 34' "4" "rechts unten" in der Nähe von Lesemodul 35 "8". Durch eine Auswertung wird bestimmt, dass sich der Benutzer mit dem Bediengerät 2 (Tag 34 "1") in der unteren rechten Ecke des Operationssaals 10' befindet und zur Raummitte bzw. in Richtung der Medizingeräte schaut. Auf dem Startbildschirm (Fig. 10) wird das Medizingerät mit dem Tag 34' "4" links, das Medizingerät mit dem Tag 34' "2 in der Mitte und das Medizingerät mit dem Tag 34' "3" rechts auf dem Bediengerät 2 angezeigt.

Ein weiteres, alternatives Verfahren enthält ein Bestimmen und Abspeichern von Signalstärken für vorbestimmte Positionen der Tags 34, 34' bzw. deren Koordinaten in dem Raum, also dem Operationssaal 10, 10'. Die Signalstärken werden an mindestens drei Lesemodulen 35 empfangen. Die vorbestimmte Position mit ihren Koordinaten wird gemeinsam mit den jeweiligen zugehörigen Signalstärken in dem medizinischen Steuerungssystem 1 in einer Datenbank abgespeichert. Es wird eine ausreichende Anzahl von vorbestimmten Positionen mit den Signalstärken abgespeichert, um eine erforderliche Genauigkeit des Systems zu gewährleisten. Im Betrieb werden für die Tags 34, 34' die jeweiligen Signalstärken an den Lesemodulen 35 erfasst und die daraus resultierenden Koordinaten aus der Datenbank für die jeweiligen Positionen der Tags 34, 34' zugeordnet.

Die verschiedenen Ausführungsformen und Alternativen des medizinischen Steuerungssystems sind kombinierbar.

## Patentansprüche

1. Medizinisches Steuerungssystem (1) mit
einem Bediengerät (2) mit einem Touchscreen (3), wobei
das Bediengerät (2) eine erste Sendeeinheit (34) aufweist, die elektromagnetische Strahlung aussendet,
einer Steuerungsvorrichtung (4), und
mehreren Medizingeräten (5, 6, 7, 8),
wobei die Medizingeräte (5, 6, 7, 8) zweite Sendeeinheiten (34') aufweisen, die elektromagnetische Strahlung aussenden, und
wobei die Medizingeräte (5, 6, 7, 8) und das Bediengerät (2) jeweils eine Datenverbindung (9) mit der Steuerungsvorrichtung (4) aufweisen,
wobei das Bediengerät (2) angepasst ist, auf dem Touchscreen (3) eine schematische Darstellung der Medizingeräte (5, 6, 7, 8) anzuzeigen,
wobei das Bediengerät (2) angepasst ist, durch Berühren einer der schematischen Darstellungen eines von den Medizingeräten (5, 6, 7, 8) eine Darstellung von spezifischen Bedienfunktionen zu ermöglichen,
wobei mehrere in einem Raum an vorbestimmten Positionen angeordnete Lesemodule (35) die von den ersten und zweiten Sendeeinheiten (34, 34') ausgesendete elektromagnetische Strahlen empfangen, wobei das medizinische Steuerungssystem (1) angepasst ist, über eine Messung von Signalstärken der von den Lesemodulen empfangenen elektromagnetischen Strahlen relative Positionen der Medizingeräte (5, 6, 7, 8) zu dem Bediengerät (2) zu bestimmen und an das Bediengerät (2) zu übermitteln, und
das Bediengerät (2) angepasst ist, die schematische Darstellung der Medizingeräte (5, 6, 7, 8) entsprechend den relativen Positionen anzuzeigen, so dass eine Anordnung von auf dem Bediengerät (2) dargestellten Positionen der Medizingeräte (5, 6, 7, 8) einer Anordnung der Medizingeräte (5, 6, 7, 8) aus einer Sicht des Bediengeräts (2) entspricht.

2. Medizinisches Steuerungssystem (1) gemäß Anspruch 1, wobei
das Bediengerät (2) angepasst ist, die schematische Darstellung der Medizingeräte (5, 6, 7, 8) auf dem Touchscreen (3) in einer ersten Anzeige (11) anzuzeigen, und
durch Berühren einer der schematischen Darstellungen von einem der Medizingeräte (5, 6, 7, 8) in der ersten Anzeige (11) eine Darstellung des einen Medizingeräts (5, 6, 7, 8) mit der Darstellung von seinen spezifischen Bedienfunktionen in einer zweiten Anzeige (14) zu ermöglichen,
wobei die Anzeigen (11, 14) für im Wesentlichen gleiche spezifische Bedienfunktionen der Medizingeräte (5, 6, 7, 8) jeweils im Wesentlichen gleiche Symbole zu deren Darstellung aufweisen,
wobei das in der zweiten Anzeige (14) dargestellte Medizingerät (5, 6, 7, 8) angetriebene Komponenten (15) aufweist, und das Bediengerät (2) angepasst ist, eine Darstellung der angetriebenen Komponenten (15) mit Symbolen (17) für spezifische Bedienfunktionen zu ermöglichen, und
wobei das Bediengerät (2) angepasst ist, durch Berühren der Darstellung von einer der angetriebenen Komponenten (15) auf dem Touchscreen (3) eine Darstellung von ihren möglichen Bewegungsrichtung (20) und Vorgaben für ihre mögliche Bewegungsgeschwindigkeit (21, 22) in einer dritten Anzeige (18) darzustellen.

3. Medizinisches Steuerungssystem (1) gemäß Anspruch 2, wobei die spezifische Bedienfunktion ein Bewegen von einer oder von mehreren der angetriebenen Komponenten (15) in eine jeweils vorbestimmte Position ist, und das Bediengerät (2) angepasst ist, durch Berühren eines Preset-Symbols (32) für das Bewegen in die vorbestimmte Position in der zweiten Anzeige (14) eine Darstellung von Endlagen der angetriebenen Komponenten (15) und von Vorgaben für eine mögliche Bewegungsgeschwindigkeit (21, 22) in einer dritten Anzeige (18) zu ermöglichen.

4. Medizinisches Steuerungssystem (1) gemäß Anspruch 3, wobei ein Abspeichersymbol (29) vorgesehen ist, und das medizinische Steuerungssystem (1) angepasst ist, durch Berühren des Abspeichersymbols (29) ein Abspeichern der jeweiligen momentanen Positionen der angetriebenen Komponenten (15) zu ermöglichen.

5. Medizinisches Steuerungssystem (1) gemäß einem der Ansprüche 2 bis 4, wobei das in der zweiten Anzeige (14) dargestellte Medizingerät (5, 6, 7, 8) Beleuchtungskomponenten (30) aufweist, und das Bediengerät (2) angepasst ist, die Beleuchtungskomponenten (30) mit einer Darstellung von Symbolen (17) für spezifische Bedienfunktionen in der zweiten Anzeige (14) zu ermöglichen.

6. Medizinisches Steuerungssystem (1) gemäß Anspruch 5, wobei das Bediengerät (2) angepasst ist, durch Berühren eines der Symbole (17) für spezifische Bedienfunktionen eine Darstellung von Symbolen (33) für einstellbare Parameter zu ermöglichen, wobei die spezifischen Bedienfunktion eine Helligkeitseinstellung und/oder eine Einstellung einer Leuchtfeldgröße und/oder eine Farbtemperatureinstellung und/oder eine Auswahl von betriebenen Beleuchtungskomponenten (30) .

7. Medizinisches Steuerungssystem (1) gemäß einem der vorangehenden Ansprüche, wobei die Medizingeräte (5, 6, 7, 8) an vordefinierten Positionen in einem Raum vorgesehen sind, und das Bediengerät (2) angepasst ist, eine Darstellung des Raums und der Medizingeräte (5, 6, 7, 8) an den vordefinierten Positionen darzustellen.

8. Medizinisches Steuerungssystem (1) gemäß einem der vorangehenden Ansprüche, wobei die ersten und zweiten Sendeeinheiten (34, 34') als Funk-Sendeeinheiten und die Lesemodule als Funk-Empfangseinheiten (35) ausgebildet sind.

9. Medizinisches Steuerungssystem (1) gemäß einem der vorangehenden Ansprüche , wobei in dem Raum drei Lesemodule (35) angeordnet sind und das medizinische Steuerungssystem (1) ausgebildet ist, die relativen Positionen des Bediengeräts (2) zu den Medizingeräten (5, 6, 7, 8) über eine Triangulation der Signalstärken zu bestimmen.

10. Medizinisches Steuerungssystem (1) gemäß einem der Ansprüche 1 bis 8, wobei in dem Raum mindestens vier Lesemodule (35) angeordnet sind und das medizinische Steuerungssystem (1) ausgebildet ist, die relativen Positionen des Bediengeräts (2) zu den Medizingeräten (5, 6, 7, 8) über eine Bereichsbestimmung zu bestimmen.

11. Medizinisches Steuerungssystem (1) gemäß einem der Ansprüche 1 bis 8, wobei in dem Raum mindestens drei Lesemodule (35) angeordnet sind und das medizinische Steuerungssystem (1) ausgebildet ist, Koordinaten von vorbestimmten Positionen des Bediengeräts (2) und der Medizingeräte (5, 6, 7, 8) in dem Raum und jeweilige zugehörige von den Lesemodulen (35) empfangene Signalstärken abzuspeichern und abzurufen, um die relativen Positionen des Bediengeräts (2) zu den Medizingeräten (5, 6, 7, 8) über das Abrufen der zu jeweiligen Signalstärken gespeicherten Koordinaten zu bestimmen.

12. Verfahren zum Betreiben eines medizinischen Steuerungssystems (1) gemäß einem der Ansprüche 1 bis 11, wobei die erste Sendeeinheit (34) und die zweiten Sendeeinheiten (34') elektromagnetische Strahlen aussenden, die Lesemodule (35) die elektromagnetischen Strahlen empfangen, und das medizinische Steuerungssystem (1) die relativen Positionen des Bediengeräts (2) zu den Medizingeräten (5, 6, 7, 8) über eine Messung der von den Lesemodulen (35) empfangenen Signalstärken bestimmt und an das Bediengerät (2) übermittelt und das Bediengerät (2) auf einem Startbildschirm die schematische Darstellung der Medizingeräte (5, 6, 7, 8) entsprechend den relativen Positionen anzeigt, so dass eine Anordnung von auf dem Bediengerät (2) dargestellten Positionen der Medizingeräte (5, 6, 7, 8) einer Anordnung der Medizingeräte (5, 6, 7, 8) aus einer Sicht des Bediengeräts (2) entspricht.

13. Verfahren gemäß Anspruch 12 mit einem medizinischen Steuerungssystem (1) gemäß Anspruch 9, wobei das medizinische Steuerungssystem (1) eine Triangulation der Signalstärken ausführt, um jeweils eine genaue Position der Sendeeinheiten (34, 34') zu bestimmen, und/oder das medizinische Steuerungssystem (1) jeweils einen Bereich eines Raums bestimmt, in dem sich die Sendeeinheiten (34, 34') befinden, wobei der Bereich des Raums jeweils einem der Lesemodule (35) zugeordnet ist, und/oder Koordinaten und Signalstärken von vorbestimmten Positionen des Bediengeräts (2) und der Medizingeräte (5, 6, 7, 8) abgespeichert werden, und den empfangenen Signalstärken resultierende abgespeicherte Koordinaten der Positionen des Bediengeräts (2) und der Medizingeräte (5, 6, 7, 8) zugeordnet werden.

## Claims

1. Medical control system (1) having
an operator device (2) having a touchscreen (3), wherein
the operator device (2) comprises a first transmitting unit (34) emitting electromagnetic radiation,
a control device (4), and
several medical apparatuses (5, 6, 7, 8),
wherein
the medical apparatuses (5, 6, 7, 8) comprise second transmitting units (34') emitting electromagnetic radiation, and
wherein
the medical apparatuses (5, 6, 7, 8) and the operator device (2) respectively comprise a data connection (9) with the control device (4),
wherein
the operator device (2) is adapted to display a schematic illustration of the medical apparatuses (5, 6, 7, 8) on the touchscreen (3),
wherein
the operator device (2) is adapted to enable an illustration of specific operation functions by touching one of the schematic illustrations of one of the medical apparatuses (5, 6, 7, 8),
wherein
several reading modules (35) arranged in a room at predefined positions receive the electromagnetic rays emitted by first and second transmitting units (34, 34'),
wherein the medical control system (1) is adapted to determine, by a measurement of signal strengths of the electromagnetic rays received by the reading modules, and to transmit to the operator device (2) relative positions of the medical apparatuses (5, 6, 7, 8) to the operator device (2), and
the operator device (2) is adapted to display the schematic illustration of the medical apparatuses (5, 6, 7, 8) according to the relative positions so that an arrangement of the positions of the medical apparatuses (5, 6, 7, 8) illustrated on the operator device (2) corresponds to an arrangement of the medical apparatuses (5, 6, 7, 8) from a view of the operator device (2).

2. Medical control system (1) according to claim 1, wherein
the operator device (2) is adapted to display the schematic illustration of the medical apparatuses (5, 6, 7, 8) in a first display (11) on the touchscreen (3), and to enable an illustration of the one medical apparatus (5, 6, 7, 8) with an illustration of its specific operation functions in a second display (14) by touching one of the schematic illustrations of one of the medical apparatuses (5, 6, 7, 8) in the first display (11),
wherein, for substantially similar specific operation functions of the medical apparatuses (5, 6, 7, 8), the displays (11, 14) comprise respectively substantially similar symbols for their illustration,
wherein the medical apparatus (5, 6, 7, 8) illustrated in the second display (14) comprises driven components (15), and the operator device (2) is adapted to enable an illustration of the driven components (15) by symbols (17) for specific operation functions, and
wherein, by touching the illustration of one of the driven components (15) on the touchscreen (3), the operator device (2) is adapted to illustrate an illustration of its possible direction of movement (20) and defaults for its possible speed of movements (21, 22) in a third display (18).

3. Medical control system (1) according to claim 2, wherein the specific operation function is a motion of one or of several of the driven components (15) into a respectively predefined position, and the operator device (2) is adapted to enable an illustration of end positions of the driven components (15) and of defaults for a possible speed of movement (21, 22) in a third display (18) by touching a preset symbol (32) for the motion into the predefined position in the second display (14).

4. Medical control system (1) according to claim 3, wherein a saving symbol (29) is provided, and the medical control system (1) is adapted to enable storing of the respective actual positions of the driven components (15) by touching the saving symbol (29).

5. Medical control system (1) according to anyone of the claims 2 to 4, wherein the medical apparatus (5, 6, 7, 8) illustrated in the second display (14) comprises lighting components (30), and the operator device (2) is adapted to enable the lighting components (30) with an illustration of symbols (17) for specific operation functions in the second display (14).

6. Medical control system (1) according to claim 5, wherein the operator device (2) is adapted to enable an illustration of symbols (33) for adjustable parameters by touching one of the symbols (17) for specific operation functions, wherein the specific operation function is a brightness setting and/or an adjustment of a size of a light field and/or an adjustment of the color temperature and/or a selection of operated lighting components (30).

7. Medical control device (1) according to anyone of the preceding claims, wherein the medical apparatuses (5, 6, 7, 8) are provided at predefined positions in a room, and the operator device (2) is adapted to illustrate an illustration of the room and of the medical apparatuses (5, 6, 7, 8) at the predefined positions.

8. Medical control device (1) according to anyone of the preceding claims, wherein the first and second transmitting units (34, 34') are designed as radio transmitting units and the reading modules are designed as radio receiving units (35).

9. Medical control device (1) according to anyone of the preceding claims, wherein three reading modules (35) are arranged in the room, and the medical control system (1) is adapted to determine the relative positions of the operator device (2) and of the medical apparatuses (5, 6, 7, 8) by means of a triangulation of the signal strengths.

10. Medical control device (1) according to anyone of the claims 1 to 8, wherein at least four reading modules (35) are arranged in the room, and the medical control system (1) is adapted to determine the relative positions of the operator device (2) to the medical apparatuses (5, 6, 7, 8) by means of an area determination.

11. Medical control device (1) according to anyone of the claims 1 to 8, wherein at least three reading modules (35) are arranged in the room, and the medical control system (1) is adapted to store and recall coordinates of predefined positions of the operator device (2) and of the medical apparatuses (5, 6, 7, 8) in the room and respective related signal strengths received by the reading modules (35) in order to determine the relative positions of the operator device (2) to the medical apparatuses (5, 6, 7, 8) by means of the recall of the coordinates stored to respective signal strengths.

12. Method for operating a medical control system (1) according to anyone of the claims 1 to 11, wherein the first transmitting unit (34) and the second transmitting unit (34') emit electromagnetic rays, the reading modules (35) receive the electromagnetic rays, and the medical control system (1) determines and transmits to the operator device (2) the relative positions of the operator device (2) to the medical apparatuses (5, 6, 7, 8) by means of a measurement of the signal strengths received by the reading modules (35), and the operator device (2) displays the schematic illustration of the medical apparatuses (5, 6, 7, 8) according to the relative positions on a start screen so that an arrangement of positions of the medical apparatuses (5, 6, 7, 8) illustrated on the operator device (2) corresponds to an arrangement of the medical apparatuses (5, 6, 7, 8) from a view of the operator device (2).

13. Method according to claim 12 with a medical control system (1) according to claim 9, wherein the medical control system (1) performs a triangulation of the signal strengths in order to determine an exact position of the transmitting units (34, 34') and/or the medical control system (1) respectively determines an area of a room in which the transmitting units (34, 34') are located, wherein the area of the room is respectively allocated to one of the reading modules (35) and/or coordinates and signal strengths of predetermined positions of the operator device (2) and of the medical apparatuses (5, 6, 7, 8) are stored, and resulting stored coordinates of the positions of the operator device (2) and the medical apparatuses (5, 6, 7, 8) are allocated to the received signal strengths.

## Revendications

1. Système de commande médical (1) comprenant un terminal de commande de service (2) avec un écran tactile (3), le terminal de commande de service (2) présentant une première unité d'émission (34), qui émet un rayonnement électromagnétique,
une unité de commande (4), et
plusieurs appareils médicaux (5, 6, 7, 8), les appareils médicaux (5, 6, 7, 8) présentant des deuxièmes unités d'émission (34'), qui émettent un rayonnement électromagnétique, et les appareils médicaux (5, 6, 7, 8) et le terminal de commande de service (2) présentant chacun respectivement une liaison de transmission de données (9) avec l'unité de commande (4),
système
dans lequel le terminal de commande de service (2) est adapté à afficher sur l'écran tactile (3), une représentation schématique des appareils médicaux (5, 6, 7, 8),
dans lequel le terminal de commande de service (2) est adapté, par effleurement de l'une des représentations schématiques de l'un des appareils médicaux (5, 6, 7, 8), à permettre une représentation de fonctions de service spécifiques,
dans lequel plusieurs modules de lecture (35) agencés dans des positions prédéterminées dans un espace ou un local, assurent la réception des rayons électromagnétiques émis par les première et deuxième unités d'émission (34, 34'),
le système de commande médical (1) étant adapté, par l'intermédiaire d'une mesure d'intensités de signal des rayons électromagnétiques reçus par les modules de lecture, à déterminer des positions relatives des appareils médicaux (5, 6, 7, 8) par rapport au terminal de commande de service (2) et à les transmettre au terminal de commande de service (2), et
le terminal de commande de service (2) étant adapté à afficher la représentation schématique des appareils médicaux (5, 6, 7, 8) conformément aux positions relatives, de sorte qu'un agencement des positions des appareils médicaux (5, 6, 7, 8) représentées sur le terminal de commande de service (2), correspond à un agencement des appareils médicaux (5, 6, 7, 8) vus à partir du terminal de commande de service (2).

2. Système de commande médical (1) selon la revendication 1,
dans lequel le terminal de commande de service (2) est adapté à afficher la représentation schématique des appareils médicaux (5, 6, 7, 8) sur l'écran tactile (3) dans un premier affichage d'écran (11), et à permettre, par effleurement de l'une des représentations schématiques de l'un des appareils médicaux (5, 6, 7, 8) sur le premier affichage d'écran (11), une représentation dudit un appareils médical (5, 6, 7, 8) considéré avec une représentation de ses fonctions de commande de service spécifiques, sur un deuxième affichage d'écran (14),
dans lequel les affichages d'écran (11, 14) pour des fonctions de commande de service spécifiques, sensiblement identiques, des appareils médicaux (5, 6, 7, 8), présentent respectivement des symboles sensiblement identiques pour leur représentation,
dans lequel l'appareil médical (5, 6, 7, 8) représenté sur le deuxième affichage d'écran (14) présente des composants entraînés (15), et le terminal de commande de service (2) est adapté à permettre une représentation des composants entraînés avec des symboles (17) pour des fonctions de commande de service spécifiques, et
dans lequel le terminal de commande de service (2) est adapté, par effleurement de la représentation de l'un des composants entraînés (15) sur l'écran tactile (3), à afficher une représentation de sa possible direction de déplacement (20) et de prescriptions pour sa possible vitesse de déplacement (21, 22), sur un troisième affichage d'écran (18).

3. Système de commande médical (1) selon la revendication 2,
dans lequel la fonction de commande de service spécifique est un déplacement d'un ou de plusieurs des composants (15) entraînés dans une position respectivement prédéterminée, et le terminal de commande de service (2) est adapté, par effleurement d'un symbole de préréglage (32) pour le déplacement dans la position prédéterminée sur le deuxième affichage d'écran (14), à permettre une représentation de positions extrêmes des composants entraînés (15) et de prescriptions pour une possible vitesse de déplacement (21, 22), sur un troisième affichage d'écran (18).

4. Système de commande médical (1) selon la revendication 3,
dans lequel il est prévu un symbole de mémorisation (29), et le système de commande médical (1) est adapté, par effleurement du symbole de mémorisation (29), à permettre une mémorisation des positions momentanées respectives des composants entraînés (15).

5. Système de commande médical (1) selon l'une des revendications 2 à 4,
dans lequel l'appareil médical (5, 6, 7, 8) représenté sur le deuxième affichage d'écran (14) présente des composants d'éclairage (30), et le terminal de commande de service (2) est adapté à permettre d'afficher les composants d'éclairage (30) avec une représentation de symboles (17) pour des fonctions de commande de service spécifiques, sur le deuxième affichage d'écran (14).

6. Système de commande médical (1) selon la revendication 5,
dans lequel le terminal de commande de service (2) est adapté, par effleurement de l'un des symboles (17) pour des fonctions de commande de service spécifiques, à permettre une représentation de symboles (33) pour des paramètres réglables, la fonction de commande de service spécifique pouvant être un réglage de luminosité et/ou un réglage d'une grandeur de champ d'éclairement et/ou un réglage de température de couleur et/ou une sélection de composants d'éclairage (30) en fonctionnement.

7. Système de commande médical (1) selon l'une des revendications précédentes,
dans lequel les appareils médicaux (5, 6, 7, 8) sont prévus dans des positions prédéfinies dans un espace ou un local, et le terminal de commande de service (2) est adapté à afficher une représentation de l'espace ou du local et des appareils médicaux (5, 6, 7, 8) aux positions prédéfinies

8. Système de commande médical (1) selon l'une des revendications précédentes,
dans lequel les première et deuxième unités d'émission (34, 34') sont réalisées en tant qu'unités d'émission radio, et les modules de lecture sont réalisés en tant qu'unités de réception radio (35).

9. Système de commande médical (1) selon l'une des revendications précédentes,
dans lequel trois modules de lecture (35) sont agencés dans ledit espace ou le local, et le système de commande médical (1) est conçu pour déterminer les positions relatives du terminal de commande de service (2) par rapport aux appareils médicaux (5, 6, 7, 8), moyennant une triangulation des intensités de signal.

10. Système de commande médical (1) selon l'une des revendications 1 à 8,
dans lequel au moins quatre modules de lecture (35) sont agencés dans ledit espace ou le local, et le système de commande médical (1) est conçu pour déterminer les positions relatives du terminal de commande de service (2) par rapport aux appareils médicaux (5, 6, 7, 8) moyennant une détermination de zone.

11. Système de commande médical (1) selon l'une des revendications 1 à 8,
dans lequel au moins trois modules de lecture (35) sont agencés dans l'espace ou le local, et le système de commande médical (1) est conçu pour mémoriser et appeler des coordonnées de positions prédéterminées du terminal de commande de service (2) et des appareils médicaux (5, 6, 7, 8) dans l'espace ou le local et des intensités de signal respectivement associées reçus par les modules de lecture (35), en vue de déterminer les positions relatives du terminal de commande de service (2) par rapport aux appareils médicaux (5, 6, 7, 8), moyennant l'appel des coordonnées mémorisées en regard des intensités de signal respectives.

12. Procédé pour assurer le fonctionnement d'un système de commande médical (1) selon l'une des revendications 1 à 11, d'après lequel la première unité d'émission (4) et les deuxièmes unités d'émission (34') émettent des rayons électromagnétiques, les modules de lecture (35) assurent la réception des rayons électromagnétiques, et le système de commande médical (1) détermine les positions relatives du terminal de commande de service (2) par rapport aux appareils médicaux (5, 6, 7, 8) moyennant une mesure des intensités de signal reçues par les modules de lecture (35), et les transmet au terminal de commande de service (2), et le terminal de commande de service (2) affiche sur un écran de démarrage, la représentation schématique des appareils médicaux (5, 6, 7, 8) conformément aux positions relatives, de sorte qu'un agencement des positions des appareils médicaux (5, 6, 7, 8) représentées sur le terminal de commande de service (2), correspond à un agencement des appareils médicaux (5, 6, 7, 8) vus à partir du terminal de commande de service (2).

13. Procédé selon la revendication 12 exécuté avec un système de commande médical (1) selon la revendication 9, d'après lequel le système de commande médical (1) effectue une triangulation des intensités de signal pour déterminer respectivement une position précise des unités d'émission (34, 34'), et/ou le système de commande médical (1) détermine respectivement une zone d'un espace ou d'un local, dans laquelle se trouvent les unités d'émission (34, 34'), la zone de l'espace ou du local étant respectivement associée à l'un des modules de lecture (35), et/ou l'on mémorise des coordonnées et des intensités de signal de positions prédéterminées du terminal de commande de service (2) et des appareils médicaux (5, 6, 7, 8), et l'on associe aux intensités de signal reçues, des coordonnées résultantes, mémorisées, des positions du terminal de commande de service (2) et des appareils médicaux (5, 6, 7, 8).
